(19) Europäisches Patentamt
European Patent Office
Office européen des brevets

(11) Veröffentlichungsnummer: **0 153 905**
**B1**

(12) # EUROPÄISCHE PATENTSCHRIFT

(45) Veröffentlichungstag der Patentschrift: **20.04.88**

(21) Anmeldenummer: **85810046.4**

(22) Anmeldetag: **07.02.85**

(51) Int. Cl.[4]: **C 07 D 345/00**, C 25 B 3/02, H 01 B 1/12, H 01 G 1/01, H 01 M 4/60, H 01 M 4/66, H 01 L 43/08, H 01 L 37/00

(54) **(2-Fluor-5,6,11,12-tetraselenotetracen)2-chlorid, Verfahren zu dessen Herstellung und dessen Verwendung.**

(30) Priorität: **13.02.84 CH 678/84**

(43) Veröffentlichungstag der Anmeldung: **04.09.85 Patentblatt 85/36**

(45) Bekanntmachung des Hinweises auf die Patenterteilung: **20.04.88 Patentblatt 88/16**

(84) Benannte Vertragsstaaten: **BE CH DE FR GB IT LI NL**

(56) Entgegenhaltungen:
**EP - A - 0 023 988**
**EP - A - 0 109 360**
**DE - A - 2 535 124**
**US - A - 3 769 276**

**SOVIET INVENTIONS ILLUSTRATED, Sektion Ch; Woche J 47, 12. Januar 1983, Derwent Publications LTD., London, L 03**

(73) Patentinhaber: **CIBA-GEIGY AG, Klybeckstrasse 141, CH-4002 Basel (CH)**

(72) Erfinder: **Hilti, Bruno, Dr., Marschalkenstrasse 27, CH-4054 Basel (CH)**
Erfinder: **Mayer, Carl W., Dr., Niederholzboden 55, CH-4125 Riehen (CH)**

**EP 0 153 905 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft das (2-Fluor-5,6,11,12-tetraselenotetracen)$_2$-chlorid, ein Verfahren zu dessen Herstellung und dessen Verwendung in Informationssystemen oder elektronischen Komponenten.

Aus der Literatur sind verschiedene metallisch leitende chalkogenierte Tetracen-Komplexe bekannt, wie z.B. das (5,6,11,12-Tetraselenotetracen)$_2$-Jodid, -Bromid oder -Chlorid oder das (5, 6, 11, 12-Tetrathiotetracen)$_2$ (Jod)$_3$. Diese Komplexe zeigen bei Temperaturen zwischen etwa 30 und 45 K einen relativ scharfen Übergang vom metallischen in den nicht-leitenden Zustand, d.h. die metallische Phase dieser Komplexe ist nicht bis zu genügend tiefen Temperaturen, bei denen z.B. Supraleitung erwartet werden kann, stabil. Es ist auch bekannt, dass der Übergangspunkt vom metallischen zum nicht-leitenden Zustand bei dem (Tetrathiotetracen)$_2$-(Jod)$_3$-Komplex unter Druck oder durch Veränderung der Stöchiometrie (Erhöhung der Jod-Konzentration über das Verhältnis 2:3 hinaus) erniedrigt werden kann. Es wird angenommen, dass die Stabilisierung der metallischen Phase bei von der exakten 2:3 Stöchiometrie abweichenden Komplexen durch eine Änderung der Bandfüllung hervorgerufen wird. Der Mechanismus, welcher unter Einwirkung von Druck bei den obigen Komplexen die Stabilisierung der metallischen Phase bewirkt, ist noch weitgehend unbekannt. Komplexe auf der Basis von 5,6,11,12-Tetrathiotetracen- bzw. tetraselenotetracen-halogeniden, die nur durch Temperaturänderung und ohne Einwirkung von Druck eine metallische Phasenumwandlung aufweisen, sind bisher noch nicht bekannt geworden.

Gegenstand vorliegender Erfindung ist ein Komplex der Formel I

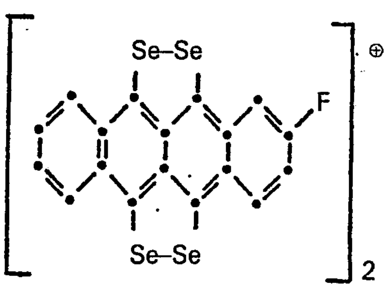

Cl$^{\ominus}$ (I)

der sich bei Normaldruck überraschenderweise und im Gegensatz z.B. zu den vorerwähnten (Tetraselenotetracen)$_2$-Jod-, -Brom- oder -Chlorkomplexen durch eine stabile metallische Phase bis mindestens 5 K auszeichnet, d.h. die elektrische Leitfähigkeit des Komplexes nimmt von Raumtemperatur (20–25 °C) bis mindestens 5°K zu. Darüber hinaus wird bei etwa 125 K ein ausgeprägter Sprung in der Zunahme der Leitfähigkeit beobachtet. Bei Raumtemperatur weist der Komplex der Formel I eine elektrische Leitfähigkeit [δ] von bis zu 1200 ohm$^{-1}$ cm$^{-1}$ und bei 5 K eine solche von 7000 ohm$^{-1}$ cm$^{-1}$ auf (gemessen längs der bevorzugten Wachstumsrichtung = Nadelachse). Bei 125 K erhöht sich die Leitfähigkeit sprunghaft um das 2,6fache.

Der erfindungsgemässe Komplex weist die Raumgruppe $P_{2/n}$ auf. Die Längen der Achsen der Elementarzelle sind: a = 1,7 492 nm, b = 0,5 159 nm, c = 1,7 486 nm. Der Komplex ist monoklin und weist neben der hohen elektrischen Leitfähigkeit auch eine ausgeprägte elektrische und optische Anisotropie auf.

Der erfindungsgemässe Komplex kann z.B. in Form von mikrokristallinen Pulvern, als amorphe Schicht, als Schicht aus Mikrokristallen, als amorphes Pulver oder in Form von Einkristallen vorliegen und als elektrischer Leiter verwendet werden.

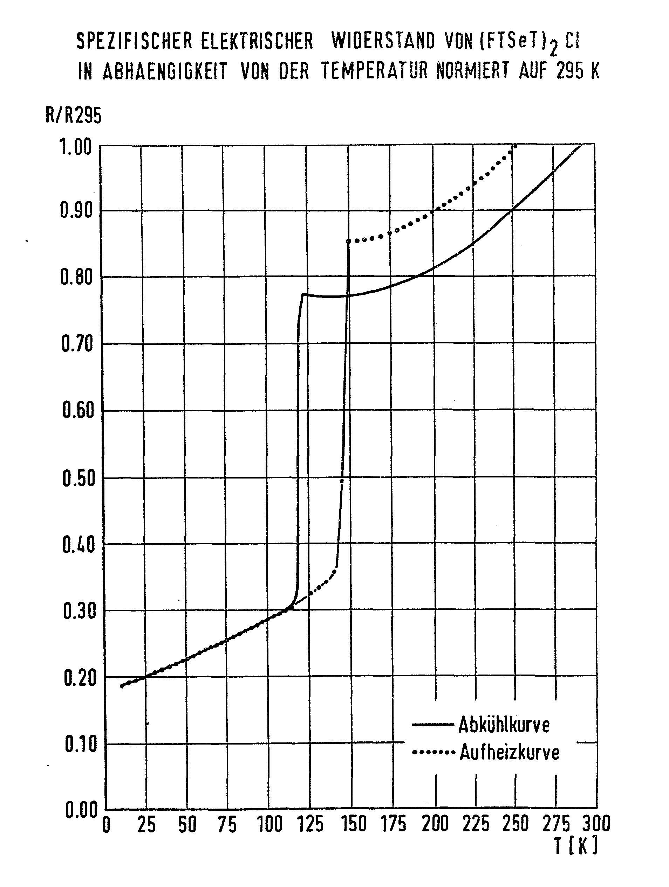

Figur 1 illustriert die Temperaturabhängigkeit des elektrischen Widerstandes des erfindungsgemässen Komplexes.

Der Komplex der Formel I kann nach verschiedenen Methoden hergestellt werden, z.B. durch (direkte) Oxidation von 2-Fluor-5,6,11,12-tetraselenotetracen mit Chlor oder einem oxidierenden, Chlorid abspaltenden Chlorsalz, wie Kupfer-(II)chlorid und $FeCl_3$, in Gegenwart eines inerten organischen Lösungsmittels. Geeignete inerte organische Lösungsmittel sind z.B. halogenierte aliphatische Kohlenwasserstoffe, wie Methylenchlorid und 1,1,2-Trichloräthan; polare substituierte, besonders halogenierte aromatische Kohlenwasserstoffe, wie Chlorbenzol, o-Dichlorbenzol, 1,2,3-Trichlorbenzol, 1,2,4-Trichlorbenzol und chlorierte Naphthaline; andere polare Lösungsmittel, wie Benzonitril und Alkylnitrile mit 2–5 C-Atomen, z.B. Acetonitril, Propionitril und Butyronitril; Nitrobenzol; N,N-Dialkylamide von aliphatischen Monocarbonsäuren mit 1–4 C-Atomen im Säureteil, z.B. N,N-Dimethylformamid und N,N-Dimethylacetamid; N,N,N',N'-Tetramethylharnstoff; Dialkylsulfoxide, wie Dimethylsulfoxid und Diäthylsulfoxid; cyclische Äther, wie Tetrahydropyran, Tetrahydrofuran und Dioxan. Es können auch Gemische der genannten Lösungsmittel eingesetzt werden. Die Reaktionstemperaturen bei diesen Oxidationsreaktionen liegen im allgemeinen zwischen 20 und 120 °C.

Der Komplex der Formel I kann auch durch Diffusion von Chlor aus der Gasphase oder aus einer Trägerlösung in eine Lösung des 2-Fluor-5,6,11, 12-tetraselenotetracens hergestellt werden, wobei als Lösungsmittel solche der oben angegebenen Art in Betracht kommen.

Der Komplex der Formel I kann ferner aus der Gasphase, d.h. durch Co-Sublimation von 2-Fluor-5,6,11,12-tetraselenotetracen und Chlor analog dem in der DE-C-26 41 742 beschriebenen Verfahren hergestellt werden. Dabei lässt man das 2-Fluor-5,6,11,12-tetraselenotetracen und das Chlor zweckmässig in einer Inertgasatmosphäre miteinander reagieren, bevorzugt in offenem System. Die Umsetzung in der Gasphase kann aber auch in geschlossenem System in Inertgasatmosphäre vorgenommen werden. Die Umsetzung in der Gasphase kann beispielsweise so erfolgen, dass man Chlorgas mittels eines inerten Trägergases mit 2-Fluor-5,6,11,12-tetraselenotetracen in der Gasphase bei ca. 260 °C in Kontakt bringt. Die

Kristalle wachsen dabei an den Reaktorwänden und/oder auf einem gegebenenfalls im Reaktor angeordneten Substrat, wie Aluminiumoxid oder bevorzugt Quarz, in beliebiger Form, z.B. in Form von Stäben oder Rohren. Als Trägergase verwendet man bei dieser Herstellungsmethode zweckmässig hochreine Inertgase, wie Argon, Stickstoff, Helium und Xenon. Die Reaktionstemperaturen bei der Gasphasenumsetzung liegen zweckmässig zwischen 180 und 300 °C. Die durch Gasphasenreaktion erhaltenen Kristalle lassen sich leicht aus der Reaktionskammer bzw. vom Substrat entfernen. Eine geeignete Versuchsanordnung für diese Herstellungsmethode ist in der oben erwähnten deutschen Patentschrift 26 41 742 beschrieben.

Bevorzugt wird der erfindungsgemässe Komplex jedoch durch elektrochemische Oxidation des 2-Fluor-5,6,11,12-tetraselenotetracens in Gegenwart eines inerten organischen Lösungsmittels und eines chloridhaltigen Leitsalzes hergestellt. Als inerte organische Lösungsmittel können solche der oben angegebenen Art eingesetzt werden. Bevorzugt sind cyclische Äther und N,N-Dialkylamide von aliphatischen Monocarbonsäuren oder Gemische davon, besonders Tetrahydrofuran und N,N-Dimethylformamid oder Gemische davon. Geeignete chloridhaltige Leitsalze sind z.B. Salze der Formel II

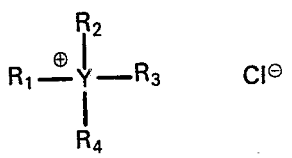 (II),

worin Y N, P oder As und $R_1$ bis $R_4$ unabhängig voneinander $C_{1-18}$-Alkyl, Benzyl, Phenyl oder Naphthyl darstellen. Alkylgruppen $R_1$ bis $R_4$ können geradkettig oder verzweigt sein und weisen bevorzugt 1–12 C-Atome auf. Als Beispiele solcher Alkylgruppen seien genannt: Methyl, Äthyl, n-Propyl, Isopropyl, n-Butyl, sek-Butyl, tert-Butyl, 1,1,3,3-Tetramethylbutyl, n-Pentyl, 2-Pentyl, n-Hexyl, n-Heptyl, n-Octyl, n-Decyl, n-Dodecyl, n-Tetradecyl, n-Hexadecyl und n-Octadecyl. Bevorzugt verwendet man Verbindungen der Formel II, worin Y N oder P, $R_1$ Benzyl oder Phenyl und $R_2$ bis $R_4$ geradkettiges Alkyl mit je 1–12 C-Atomen oder Phenyl oder $R_1$ und $R_4$ geradkettiges Alkyl mit je 1–12 C-Atomen bedeuten. Besonders bevorzugt verwendet man Verbindungen der Formel II, worin Y N und $R_1$ bis $R_4$ geradkettiges Alkyl mit je 1–12 C-Atomen und insbesondere je n-Hexyl bedeuten.

Je nach Temperatur der Elektrolysezelle und eingesetztem Lösungsmittel verwendet man zweckmässig zwischen 0,01 bis 30 g Leitsalz pro Liter. Als Elektrolysezellen können an sich bekannte Vorrichtungen verwendet werden, z.B. solche, worin der Anodenraum durch Teflonsiebe, Glasfritten oder Kapillaren vom Kathodenraum getrennt ist. Die Dimensionen der Elektrolysezellen können in Abhängigkeit der verwendeten Menge an Reaktionskomponenten variieren, beeinträchtigen jedoch die Qualität des erhaltenen Komplexes der Formel I praktisch nicht. Für die Herstellung von etwa 5–50 mg Komplex der Formel I sind z.B. Zellvolumen von 15–100 ml besonders geeignet.

Die Reaktionstemperaturen (Temperatur der Elektrolysezellen) liegen je nach Art des verwendeten Lösungsmittels mit Vorteil zwischen 0 und 120 °C. Die Stromstärke variiert im allgemeinen zwischen 0,005 µA und 5 µA. Der Durchmesser der Anoden und Kathoden beträgt zweckmässig zwischen 0,1 bis 5 mm.

Bei den obigen Umsetzungen werden das 2-Fluor-5,6,11,12-tetraselenotetracen und das Chlor oder Chloridsalz in mindestens stöchiometrischer Menge eingesetzt. Im allgemeinen empfiehlt es sich jedoch, mit einem Überschuss an Chlor oder Chloridsalz zu beginnen, so dass sich in der Reaktionsphase zu jeder Zeit ein 20- bis 1000facher molarer Überschuss an Chlor oder Chloridsalz befindet.

2-(Fluor)-5,6,11,12-tetraselenotetracen (Formel VI) ist über die Zwischenprodukte der Formeln III bis V erhältlich,

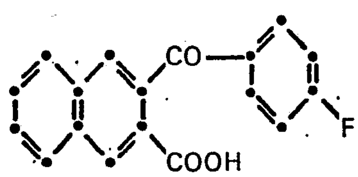 (III),

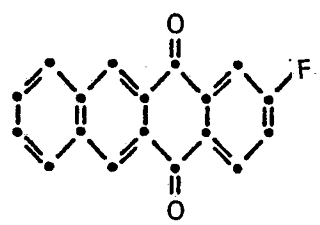 (IV),

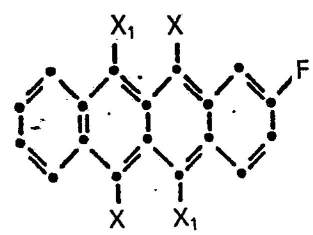 (V) und

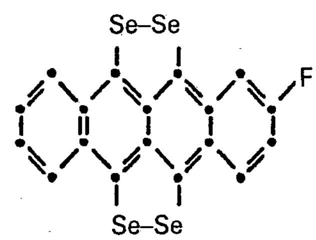 (VI),

worin die $X_1$ und X je Wasserstoff oder die $X_1$ je Wasserstoff und die X je Chlor oder die $X_1$ je Chlor und die X je Wasserstoff bedeuten.

Die Verbindungen der Formeln III bis VI können in Analogie zu an sich bekannten Verfahren dadurch hergestellt werden, dass man 2,3-Naphthalindicarbonsäureanhydrid in Gegenwart eines Friedel-Crafts-Katalysators, bevorzugt Aluminiumchlorid, mit Fluorbenzol zu einer Verbindung der Formel III umsetzt, die 2-(4-Fluorbenzoyl)-naphthalin-3-carbonsäure zum 2-Fluor-5,12-naphthacenchinon (Verbindung der Formel IV) cyclisiert, die Verbindung der Formel IV zum 2-Fluortetracen (Verbindung der Formel V mit $X_1$ und X = H) reduziert, z.B. in Gegenwart von Zinkstaub in saurem Medium, wie z.B. Essigsäure, das 2-Fluortetracen mit Sulfurylchlorid zum 2-Fluor-5,11- bzw. 2-Fluor-6,12-dichlortetracen umsetzt [vgl. z.B. Bull. Soc. Chim. France, 427 (1948)], und schliesslich das 2-Fluor-5,11- bzw. 2-Fluor-6,12-dichlortetracen oder deren Gemisch bei erhöhter Temperatur mit Selen umsetzt.

Die Cyclisierung der 2-(3-Fluorbenzoyl)-naphthalin-3-carbonsäure zum 2-Fluor-5,12-naphthacenchinon kann in an sich bekannter Weise in Gegenwart einer Protonensäure oder einer Lewis-Säure vorgenommen werden. Beispiele geeigneter Protonensäuren sind Polyphosphorsäure, Chlorsulfonsäure und Schwefelsäure. Als Lewis-Säuren kommen z.B. Bortrifluorid und vor allem Aluminiumtrichlorid in Betracht. Bevorzugt ist die Cyclisierung in Gegenwart einer Lewis-Säure, besonders Aluminiumchlorid, in der Schmelze. Die Umsetzung des 5-Fluortetracens mit dem Sulfurylchlorid sowie die Umsetzung des 2-Fluor-5,11- bzw. 2-Fluor-6,12-dichlortetracens mit dem Selen werden zweckmässig in Gegenwart eines inerten organischen Lösungsmittels vorgenommen. Für die Umsetzung des 2-Fluortetracens mit dem Sulfurylchlorid eignen sich z.B. Nitrobenzol, Benzol und Tetrachlorkohlenstoff. Bevorzugtes Lösungsmittel ist Nitrobenzol.

Die Umsetzung des 2-Fluor-5,11- bzw. 2-Fluor-6,12-dichlortetracens wird bevorzugt in Gegenwart eines halogenierten aromatischen Kohlenwasserstoffs, insbesondere 1,2,4-Trichlorbenzol, vorgenommen.

Auf Grund der metallisch elektrischen Leitfähigkeit und der ausgeprägten elektrischen und optischen Anisotropie eignet sich der erfindungsgemässe Komplex zur Verwendung als organischer elektrischer Leiter, z.B. für leitende Beschichtungen auf Kunststoffasern; des weiteren als Polarisatormaterial oder als Zusatz zu antistatischen Beschichtungen und Belägen, beispielsweise solchen auf Kunststoff-Basis. Der Komplex der Formel I kann auch in hochleitenden, elektronenstrahl- oder photoempfindlichen Druckmaterialien oder -verfahren eingesetzt werden, wie sie z.B. in der EP-A-0 023 988 und der US-A-4 036 648 beschrieben sind. Dank seiner Redoxeigenschaften und der verschiedenen intensiven Farben seiner Redoxstufen (grün, blaugrün, blau, gelb) kann der Komplex der Formel I ferner mit Vorteil in Informationssystemen, wie Farbbildschirmen, sowie in elektronischen Komponenten verwendet werden. Der hochleitende Komplex der Formel I ist hierzu besonders geeignet, da er weiterer Oxidation und Reduktion in elektrischen Anordnungen, wie sie z.B. elektrochrome Schaltungen darstellen, unterworfen werden kann. Vor allem eignet sich der erfindungsgemässe Komplex jedoch auf Grund seiner bis zu ca. 5 K stabilen metallischen Phase für verschiedene Anwendungen in der Tieftemperaturtechnolgie, z.B. zur Verwendung als elektrisch leitende Schicht in Kondensatorfolien oder als Kathodenmaterial in Festelektrolytzellen, deren Gebrauch damit auch bei tiefer Temperatur möglich ist. Ferner kann der Komplex für druck- und/oder temperaturabhängige Schaltelemente oder für magnetfeldabhängige Schaltelemente eingesetzt werden.

Beispiel

a) 45,0 g (227 mMol) 2,3-Naphthalindicarbonsäureanhydrid werden in 200 ml (2,1 Mol) Fluorbenzol suspendiert. Innerhalb von 5 Minuten werden unter starkem Rühren 75,5 g (565 mMol) pulverisiertes Aluminiumchlorid zugegeben (exotherme Reaktion bis ca. 32 °C). Die dunkelrote Suspension wird 6 Stunden am Rückfluss erhitzt und gerührt. Dann lässt man die Lösung auf Raumtemperatur abkühlen, giesst sie auf ca. 500 g Eis und rührt bis zur Vervollständigung der Hydrolyse. Überschüssiges Fluorbenzol wird abgedampft, und das erhaltene Produkt wird in Wasser suspendiert. Die Suspension wird abfiltriert, das Filtrat mit Wasser nachgewaschen und das Produkt getrocknet. Man erhält 76 g rohe 2-(4-Fluorbenzoyl)-naphthalin-3-carbonsäure. Das Rohprodukt wird mit 2 Liter 10%iger Sodalösung verrührt, die erhaltene Lösung wird mit Salzsäure angesäuert, und der weisse Niederschlag wird abfiltriert und getrocknet. Man erhält 54,2 g (81% d.Th.) 2-(4-Fluorbenzoyl)-naphthalin-3-carbonsäure.

MS: ($M^+$ = 294; $M^+$–COOH = 249, $M^+$ –COO = 250, $M^+$ –$C_6H_4F$ = 199); IR-Spektrum (KBr): OH ca. 3300 $cm^{-1}$; C = O-Doppelbande 1695/1705 $cm^{-1}$.

b) 200 g (1,5 Mol) pulverisiertes Aluminiumchlorid und 40 g (684 mMol) Natriumchlorid werden zusammen auf 140–150 °C erhitzt. Nach etwa 1,5 Stunden werden 40 g (136 mMol) 2-(4-Fluorbenzoyl)-naphthalin-3-carbonsäure zur Schmelze gegeben. Das dunkelrote Gemisch wird eine Stunde bei 140–150 °C gerührt. Anschliessend wird bei etwa 100 °C durch langsame Zugabe von Eiswasser hydrolysiert. Der Niederschlag wird abfiltriert und mit 10%iger Sodalösung verrührt. Dann wird mit Wasser neutral gewaschen und getrocknet. Das erhaltene Produkt wird bei 230 °C sublimiert. Man erhält 13,1 g (70% d.Th.) 2-Fluor-5,12-naphthacenchinon.

DC: Kieselgel, Benzol: $R_x \simeq 0,6$; gelb fluoreszierender Fleck.

NMR (100 MHz in $CDCl_3$): komplizierte, aber interpretierbare Multipletts im Aromatenbereich.

MS: $M^+$ = 276, $M^+$ –CO = 248, $M^+$ –2CO = 220.

c) 8,0 g (29 mMol) 2-Fluor-5,12-naphthacenchinon, 40 ml Wasser, 680 ml Essigsäure und 40 g (611 mMol) Zinkstaub werden zusammengegeben und zum Rückfluss erhitzt. Nach 30 Minuten Rühren am Rückfluss wird abgekühlt, und es werden 200 ml Wasser zugegeben. Die entstandene Suspension des 2-Fluortetracens wird abdekantiert, wobei der Zinkstaub im Kolben zurückbleibt. Das 2-Fluortetracen wird abfiltriert, mit Wasser und Äthanol gewaschen und getrocknet. Nach dem Umkristallisieren aus 500 ml Xylol erhält man 4,0 g (56% d.Th.) 2-Fluortetracen.

UV (Benzol): typisches Tetracenspektrum. $\lambda_{max}$ 476, 446, 420, 394 nm.

MS: $M^+ = 246$, $M^{+2} = 123$.

d) 5,0 g (20,3 mMol) 2-Fluortetracen werden unter Stickstoff in 25 ml Nitrobenzol suspendiert und auf 5 °C abgekühlt. Dann lässt man innerhalb von 30 Minuten 5,9 g (43,7 mMol) Sulfurylchlorid in 25 ml Nitrobenzol zutropfen und rührt 2 Stunden bei 5 °C. Dann lässt man die Temperatur auf 20–25 °C ansteigen, heizt innerhalb von 1,5 Stunden auf 90 °C, rührt 10 Minuten bei dieser Temperatur und kühlt ab. Die Suspension wird abfiltriert, mit ca. 600 ml Äthanol nachgewaschen und getrocknet. Man erhält 5,0 g (78% d.Th.) 2-Fluor-5,11- bzw. 6,12-dichlortetracen.

MS: $M^+ = 314/316$ (= 2 Cl), $M^+ - HCl = 278$, $M^+ - 2Cl = 244$, $M^{+2} = 157/158$ (2 Cl).

e) 7,2 g (22,8 mMol) 2-Fluor-5,11-dichlortetracen, 7,7 g (97,5 mMol) Selen und 175 ml Trichlorbenzol werden zusammengegeben und 120 Stunden bei einer Badtemperatur von 250 °C unter Stickstoff am Rückfluss gekocht. Nach 70 Stunden gibt man nochmals 3,8 g (48,1 mMol) Selen zu. Dann lässt man die Suspension abkühlen, verdünnt mit ca. 200 ml n-Hexan und filtriert ab. Das Produkt wird mit Benzol und n-Hexan nachgewaschen und getrocknet und anschliessend am Hochvakuum bei 260–270 °C/$10^{-3}$ bar sublimiert. Man erhält 4,4 g (35% d.Th.) 2-Fluor-5,6,11,12-tetraselenotetracen.

UV-Spektrum in Trichlorbenzol $\lambda_{max}$ = 719, 659, 466 nm.

f) 30 mg 2-Fluor-5,6,11,12-tetraselenotetracen werden in den Anodenraum einer Elektrolysenzelle mit einem Volumen von 40 ml eingefüllt. Als Leitsalz werden 60 mg Tetra-n-hexyl-ammoniumchlorid zugesetzt. Die Zelle wird über Nacht in einem Trockenschrank bei $5 \times 10^{-2}$ mbar evakuiert und mit Argon gespült. Dann werden als Lösungsmittel 33 ml eines Gemisches auf 25 Vol.-% Chlorbenzol und 75 Vol.-% N,N-Dimethylformamid zugegeben. Nach 4 Stunden Aufheizen auf 90 °C wird die Zelle während 1 Stunde unter 0,1 Volt Spannung gesetzt; diese Spannung wird im Verlauf von 2 Tage in 3 Stufen auf 0,4 Volt erhöht, wobei sich ein Elektrolysestrom von 1,06 μA einstellt. Nach 8 Tagen werden die an der Anode (Durchmesser 1 mm; 80 Gew.-% Pt, 20 Gew.-% Ir) gebildeten Kristalle durch Abwaschen mit Äthanol abgelöst. Vier Kristalle mit den durchschnittlichen Abmessungen 4 mm × 40 μm × 40 μm werden mittels Platinpaste (Pt-Paste 308 der Fa. Degussa) auf 4 Sonden montiert, die aus 25 μm dicken Golddrähten bestehen. Die Leitfähigkeit der Kristalle bei Raumtemperatur, gemessen in der obigen Sondenanordnung variiert von 700 bis 1200 $ohm^{-1} cm^{-1}$. Die Temperaturabhängigkeit des spezifischen Widerstands, normiert auf 295 K, zeigt für alle Kristalle innerhalb einer Messgenauigkeit von 2% das in Figur 1 dargestellte Verhalten.

## Patentansprüche

1. Komplex der Formel I

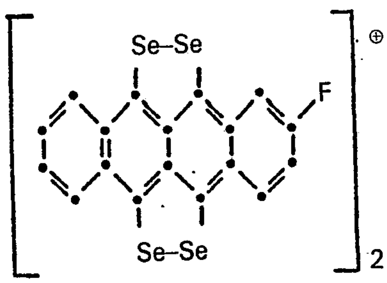

$Cl^{\ominus}$ (I).

2. Verfahren zur Herstellung des Komplexes der Formel I nach Anspruch 1, dadurch gekennzeichnet, dass man 2-Fluor-5,6,11,12-tetraselenotetracen mit Chlor oder mit einem oxidierenden, Chlorid abspaltenden Chlorsalz in Gegenwart eines inerten Lösungsmittels oxidiert.

3. Verfahren nach Anspruch 2, dadurch gekennzeichnet, dass man die Oxidation mit Chlor in der Gasphase vornimmt.

4. Verfahren nach Anspruch 2, dadurch gekennzeichnet, dass man die Oxidation elektrochemisch in Gegenwart eines chloridhaltigen Leitsalzes in einem inerten Lösungsmittel durchführt.

5. Verfahren nach Anspruch 4, dadurch gekennzeichnet, dass man als Leitsalz eine Verbindung der Formel II

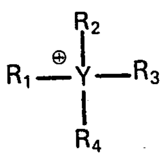

$Cl^{\ominus}$ (II)

verwendet, worin Y N, P oder As und $R_1$ bis $R_4$ unabhängig voneinander $C_{1-18}$-Alkyl, Benzyl, Phenyl oder Naphthyl darstellen.

6. Verfahren nach Anspruch 5, dadurch gekennzeichnet, dass man eine Verbindung der Formel II verwendet, worin Y N oder P, $R_1$ Benzyl, oder Phenyl und $R_2$ bis $R_4$ geradkettiges Alkyl mit je 1–12 C-Atomen, oder Phenyl oder $R_1$ bis $R_4$ geradkettiges Alkyl mit je 1–12 C-Atomen darstellen.

7. Verfahren nach Anspruch 5, dadurch gekennzeichnet, dass man eine Verbindung der Formel II verwendet, worin Y N und $R_1$ bis $R_4$ geradkettiges Alkyl mit je 1–12 C-Atomen, besonders je n-Hexyl darstellen.

8. Verwendung des Komplexes der Formel I nach Anspruch 1 in Informationssystem oder elektronischen Komponenten.

9. Verwendung des Komplexes der Formel I nach Anspruch 1 für druck- und/oder temperaturabhängige Schaltelemente oder für magnetfeldabhängige Schaltelemente.

10. Verwendung des Komplexes der Formel I nach Anspruch 1 als elektrisch leitende Schicht in Kondensatorfolien oder aktiven Batterieelektroden.

**Revendications**

1. Complexe de formule I:

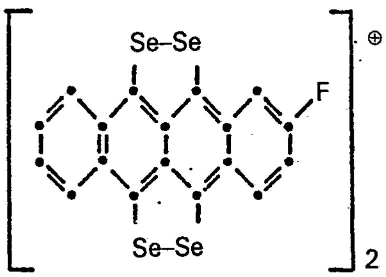

$Cl^{\ominus}$ (I).

2. Procédé pour préparer le complexe de formule I selon la revendication 1, procédé caractérisé en ce qu'on oxyde le fluoro-2 tétrasélé-no-5,6,11,12 tétracène par le chlore ou par un sel chloré oxydant libérant du chlore, en présence d'un solvant inerte.

3. Procédé selon la revendication 2, caractérisé en ce qu'on effectue l'oxydation par le chlore en phase gazeuse.

4. Procédé selon la revendication 2, caractérisé en ce qu'on effectue l'oxydation par voie électrochimique en présence d'un sel conducteur contenant un ion chlorure, dans un solvant inerte.

5. Procédé selon la revendication 4, caractérisé en ce qu'on utilise, comme sel conducteur, un composé répondant à la formule II:

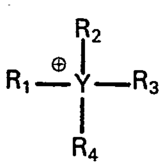

$Cl^{\ominus}$ (II)

dans laquelle Y représente N, P ou As, et $R_1$ à $R_4$ représentent chacun, indépendamment les uns des autres, un alkyle en $C_1$–$C_{18}$, un benzyle, un phényle ou un naphthyle.

6. Procédé selon la revendication 5, caractérisé en ce qu'on utilise un composé de formule II dans lequel Y représente N ou P, $R_1$ représente un benzyle ou un phényle, et $R_2$ à $R_4$ représentent chacun, indépendamment les uns des autres, un alkyle contenant de 1 à 12 atomes de carbone ou un phényle, ou encore $R_1$ à $R_4$ représentent chacun un alkyle linéaire contenant de 1 à 12 atomes de carbone.

7. Procédé selon la revendication 5, caractérisé en ce qu'on utilise un composé de formule II dans lequel Y représente N, et $R_1$ à $R_4$ représentent chacun un alkyle linéaire contenant de 1 à 12 atomes de carbone, plus particulièrement chacun un radical n-hexyle.

8. Application du complexe de formule I selon la revendication 1 dans un système d'information ou dans des composants électroniques.

9. Application du complexe de formule I selon la revendication 1 pour des éléments de circuit dépendant de la pression et/ou de la température ou pour des éléments de circuit dépendant d'un champ magnétique.

10. Application du complexe de formule I selon la revendication 1 comme couche électroconductrice dans des feuilles de condensateurs ou dans des électrodes actives de batteries.

**Claims**

1. The complex of the formula I

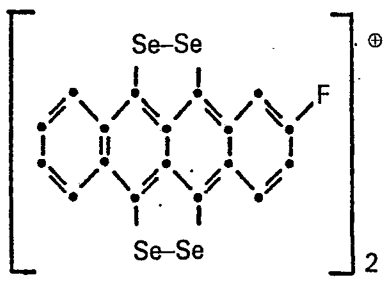

$Cl^{\ominus}$ (I).

2. A process for the preparation of the complex of the formula I according to Claim 1, which comprises oxidising 2-fluoro-5,6,11,12-tetraselenotetracene with chlorine or with an oxidising chlorine salt which splits off chloride, in the presence of an inert solvent.

3. The process according to Claim 2, wherein the oxidation is carried out with chlorine in the gas phase.

4. The process according to Claim 2, wherein the oxidation is carried out electrochemically in the presence of a chloride-containing conductive salt in an inert solvent.

5. The process according to Claim 4, wherein a compound of the formula II

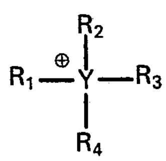

$Cl^{\ominus}$ (II)

in which Y is N, P or As and $R_1$ to $R_4$ independently of one another are $C_{1-18}$-alkyl, benzyl, phenyl or naphthyl, is used as the conductive salt.

6. The process according to Claim 5, wherein a compound of the formula II in which Y is N or P, $R_1$ is benzyl or phenyl and $R_2$ to $R_4$ are straight-chain alkyl with in each case 1–12 C-atoms or phenyl, or $R_1$ to $R_4$ are straight-chain alkyl with in each case 1–12 C-atoms, is used.

7. The process according to Claim 5, wherein a compound of the formula II in which Y is N and $R_1$ to $R_4$ are straight-chain alkyl with in each case 1–12 C-atoms, in particular in each case n-hexyl, is used.

8. The use of the complex of the formula I according to Claim 1 in information systems or electronic components.

9. The use of the complex of the formula I according to Claim 1 for pressure- and/or temperature-dependent circuit elements or for circuit elements which depend on a magnetic field.

10. The use of the complex of the formula I according to Claim 1 as an electrically conductive layer in capacitor films or active battery electrodes.

SPEZIFISCHER ELEKTRISCHER WIDERSTAND VON $(FTSeT)_2$ Cl
IN ABHAENGIGKEIT VON DER TEMPERATUR NORMIERT AUF 295 K